# EUROPEAN PATENT SPECIFICATION

(11) **EP 3 960 133 B1**
(45) Date of publication and mention of the grant of the patent: **16.08.2023**
(21) Application number: 21202926.8
(22) Date of filing: 06.09.2017
(51) Int. Cl.: A61F 5/14, A43B 7/14, A43B 17/02

(54) **AN ORTHOTIC PRODUCT**
ORTHESEPRODUKT
PRODUIT ORTHÉTIQUE

(30) Priority: 06.09.2016 AU 2016903571
(43) Date of publication of application: 02.03.2022
(62) Divisional of application: 17847804.6
(73) Proprietor: Kinetic Orthotics Pty Ltd, Marcoola, Queensland 4564 (AU)
(72) Inventor: EVERSON, Dan, Marcoola, Queensland 4564 (AU)
(74) Representative: Page, White & Farrer Germany LLP

(56) References cited:
- WO-A1-2008/031616
- AU-A- 7 566 181
- DE-C- 729 571
- DE-U1- 20 312 453
- DE-U1-202004 017 385
- US-A- 2 409 960
- US-A1- 2005 166 425
- US-A1- 2009 188 129
- US-A1- 2015 143 713
- US-A1- 2015 272 273

## Description

### Field of the invention

The present invention relates to orthotics for a foot.

### Background of the invention

Orthotics are used in the treatment of foot disorders. Such orthotics are often provided as an insert that can be placed within the patient's shoes.

Many existing orthotics consider only the contour of the foot and the position of the foot when sitting or standing.

It is only recently that developments in orthotics have begun to consider other aspects of the foot, such as the position and movement of the foot in locomotion. For example, the applicant's own Australian Patent Application No. 2012262646 describes a process in which an individual is assessed using a number of functional tests. The results of those tests can then be used to fit the individual with an orthotic that can further improve the efficiency of the individual's gait cycle and therefore mobility.

Further, AU 75 661/81 A and US 2,409,960 A each disclose an orthotic for a foot, having a top side and a bottom side, the orthotic being less than half the length of the foot, wherein the top side of the orthotic includes a raised section located at a position corresponding to the medial side of the calcaneus of the foot.

It is an object of this invention to provide an orthotic for a foot that has advantages over the prior art, or at least provides a useful alternative.

### Summary of the invention

This invention has come about due to the realisation of the inventor of the advantages of stimulating or blocking stimulation of certain parts of the foot, by means of targeted sections of an orthotic, to improve the movement pathway of an individual by improving the firing of the neuromuscular complex.

The subject-matter of the invention is defined by the appended independent claim, while some advantageous embodiments are defined in the dependent claims. The orthotic for a foot, having a top side and a bottom side, is less than half the length of the foot, wherein the top side of the orthotic includes a first raised section located at a position corresponding to the medial side of the calcaneus of the foot. According to the invention, this first raised section is also located around the insertion site of the tibialis posterior into the side of the navicular of the foot, thereby increasing the support of the orthotic to facilitate the tibialis posterior muscle group. And according to the invention, the orthotic further comprises a second raised section in the top side of the orthotic located at a position corresponding to the cuboid area of the lateral side of the foot and extending anterior along one third of the shaft of the fifth metatarsal, thereby stimulating the peroneal muscle insertions and tendons that track through this area.

The first raised section may be a substantially semicircular arc with a diameter of 2 to 4 cm.

The orthotic may further comprise an indentation in the top side of the orthotic located at a position corresponding to the base of the first metatarsal of a foot.

The orthotic may further comprise a raised section located at a position corresponding to the sulci of the foot.

The orthotic may further comprise a raised section located at a position corresponding to 2mm to 2cm behind the second to fifth metatarsal heads of a foot.

Further aspects of the claimed invention as well as further aspects being not part of the claimed invention will become apparent from the embodiments described in the detailed description and drawings.

A detailed description of one or more embodiments of the invention is provided below, along with accompanying figures that illustrate by way of example the principles of the invention.

For the purpose of example, numerous specific details are set forth in the following description in order to provide a thorough understanding of the present invention. The present invention may be practiced according to the claims without some or all of these specific details. For the purposes of clarity, technical material that is known in the technical fields related to the invention has not been described in detail so that the present invention is not unnecessarily obscured.

### Brief Description of Drawings

Various embodiments/aspects of the invention will now be described with reference to the following drawings in which:

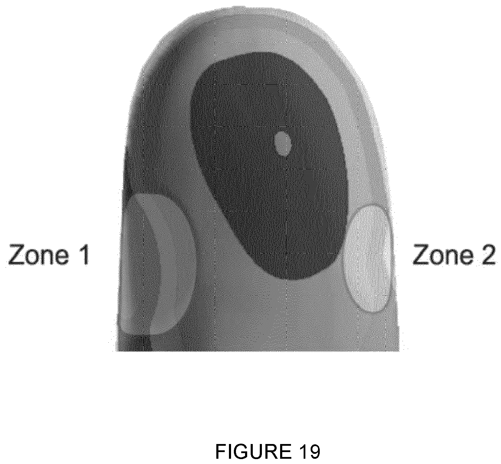

Figure 19 is a bottom view of an orthotic for a foot in accordance with an embodiment of the claimed invention.

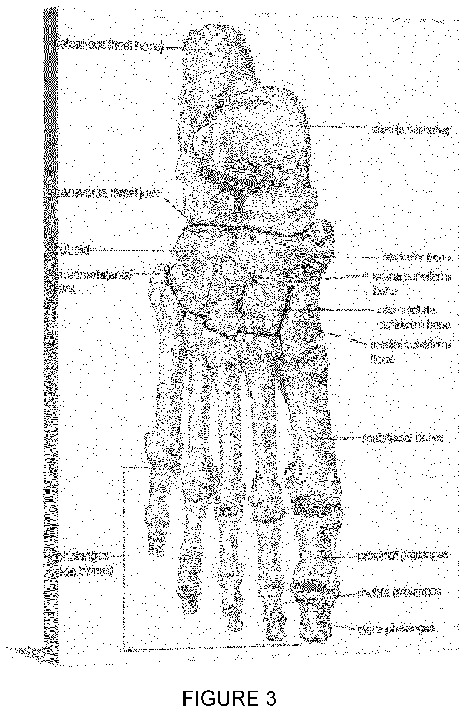

Figure 3 is a diagram of the bones of a foot.

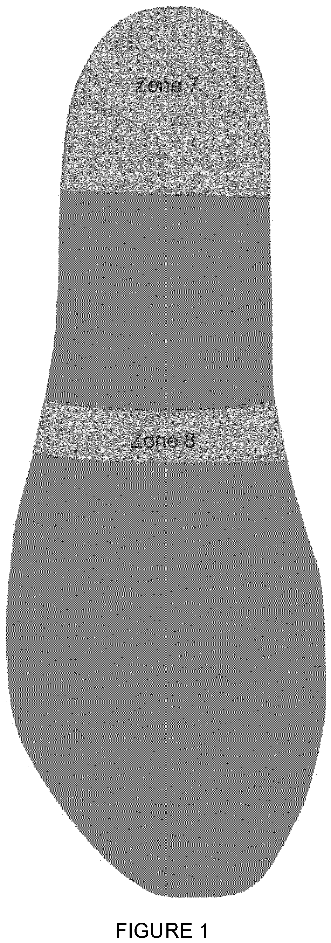

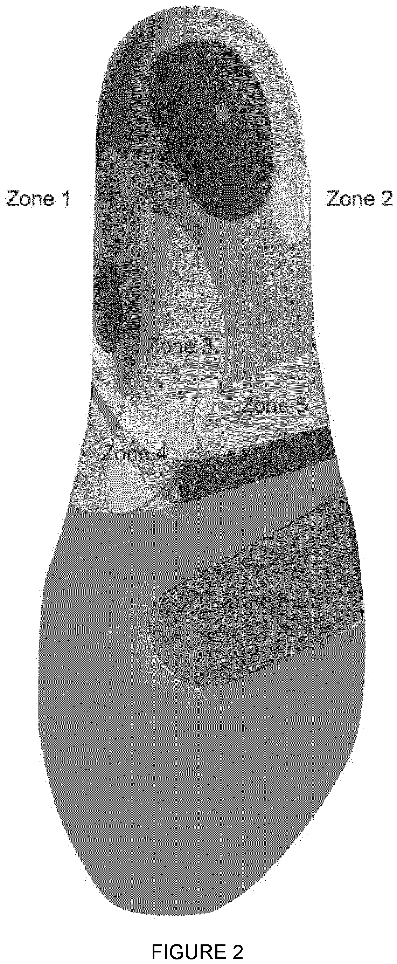

Figures 1, 2, 4 to 18 depict alternative orthotics being not part of the claimed invention, but showing some aspects being applicable in the orthotic for a foot shown in Figure 19.

### Detailed Description

The present invention will now be described with reference to one embodiment of the invention.

In Figure 19, there is depicted an orthotic having particular application in ballet, and other environments where small footwear worn such as in sprinting, cycling, or fashion. The orthotic in this embodiment is less than half length, and so takes up little room within the shoes and only has the first (calcaneal) and second (cuboid) "bumps" (ie raised sections). At least for a ballet application, no or minimal pitched heel may be used on the bottom of the orthotic. However, by stimulating of the foot using these bumps in zones 1 and 2, improved performance may be achieved in these applications.

The applicant has found that the stimulation or protection from stimulation, as appropriate, of particular locations of the foot corresponding to selected zone(s) improves the muscle function in order to achieve a more efficient gait cycle. Functional optimisation is achieved by improving the functional position of the foot. Firstly, by protecting and facilitating the windlass effect in conjunction with optimising the pivotal function at the key sagittal plane pivot sites in the foot; namely the heel, the ankle and the fore foot. Secondly, by stabilising the foot in the frontal plane in such a way as to create a functional equilibrium between the ability to efficiently pivot and the amount of support required (both in position and amplitude) to achieve this outcome.

A first zone is located in a position corresponding to the anterior of the medial side of the calcaneus and around the insertion site of the tibialis posterior into the side of the navicular of the foot. The zone is generally a substantially semicircular arc with a diameter that may vary from about 2cm to about 4cm. In an embodiment of the present invention there is provided an orthotic with a raised section in this zone, which increases the support of the orthotic to facilitate the tibialis posterior muscle group. It should be noted that, for some feet, the raised section in this zone may be slightly longer (longitudinally) than shown in the figures.

A second zone is located in a position corresponding to the area of the foot around the cuboid area of the lateral side of the foot and extending anterior along one third of the shaft of the fifth metatarsal head. In an embodiment of the present invention, a raised section is located in this zone. The stimulation of the foot by this raised zone works to stimulate the peroneal muscle insertions and tendons that track through this area. These insertions and tendons play an important role in stabilising the fore foot and the windlass effect during the propulsion phase of the gait cycle.

For each raised surface, the height of the raise is preferably greatest towards the centre of the respective zone - that is, there is a peak towards the centre of the zone. Each peak may be raised between 1mm and 25mm from the surrounding orthotic surface, but is preferably between 2mm and 20mm. and in most circumstances will be preferably between 3mm and 8mm.

Figures 1 and 2 show the top side and bottom side of another orthotic not belonging to the invention, being a full length orthotic and having eight numbered zones, i.e. six zones in addition to first and second zones shown in Figure 19.

A third zone is a fascial groove zone starting in a location corresponding to the medial tubercle of the calcaneus to its insertion point into the base of the first metatarsal. The corresponding area on a foot is responsible for protecting the windlass effect. In an embodiment of the present invention, a groove is located in this zone. By having a groove in this zone, the pressure on the fascial chord of the foot can be minimised to reduce the incidence of uneven pressure on the chord interfering with the windlass mechanism.

A fourth functional zone is located at a position corresponding to the base of the first metatarsal zone and extending across approximately 1/3 of the width of the orthotic at the metatarsal area and one fifth of the length to the posterior heel point of the orthotic length on the medial side of the orthotic. In an embodiment of the invention, there is an indentation in the orthotic in this zone. This decrease in bulk behind the first metatarsal resulting from the indentation encourages the plantar flexion of the first metatarsal.

The fifth zone is positioned at a location corresponding to 2mm to 2cm behind the second to fifth metatarsal heads on a foot. In an embodiment of the present invention, there is a raised surface in this zone. This raised surface increases the tension on the digital muscles in the foot as they insert into the metatarsal heads.

The sixth zone is located at a position corresponding to the sulci of a foot. In an embodiment of the present invention, there is a raised surface in this zone. The raised surface engages with the foot to enable better digital traction and reduce clawing of the toes. The raised section in the sixth zone may provide a wedging effect - for example, it may be higher on the inside or outside of the foot, to improve the forefoot position. Increasing the height of the raised section on the inside of the foot can be used to invert the forefoot position, or vice versa. This function of wedging the ball of the foot is quite different to the use of wedges in ordinary orthotics, where the is positioned behind the metatarsal. Note that, although (in the figures) the zone is shown as only extending about halfway laterally across the orthotic, for some people (with more severe forefoot position issues) the raised surface may be extended all the way across the orthotic.

The seventh zone is located on the bottom of the orthotic of the present invention in a position corresponding to the plantar heel surface of the foot. In an embodiment of the present invention, this zone is pitched from the rear of the orthotic to the front of the heel section. The pitched heel raise facilitates angle joint dorsiflexion.

The eighth zone is located on the bottom of the orthotic in a position corresponding to the location between the metatarsal phalangeal joints of the foot to 25mm behind the phalangeal joints, and extends from the medial to the lateral side of the orthotic. In an embodiment of the present invention, an indentation extends across this entire zone. The presence of this indentation in the orthotic improves the rocker function in the forefoot to improve the efficiency of the gait of an individual and reduce the soft tissue stress on the foot. Although the feature has been described above as an indentation, in other embodiments it may simply be a flexion zone or hinge point, defined by a weakness or other arrangement (for example use of a more resilient material in the orthotic).

Orthotics may be pre-made with the raised surfaces or grooves in all or most of the above-designed zones as it is anticipated that almost all patients in need of orthotics would benefit from stimulation/blocking of stimulation (as appropriate) in the identified zones.

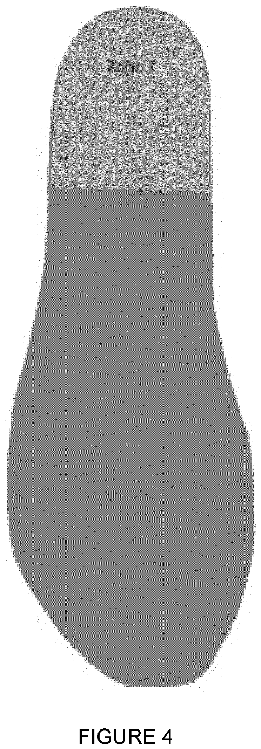

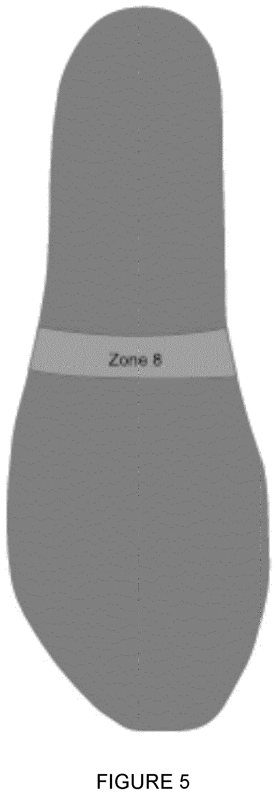

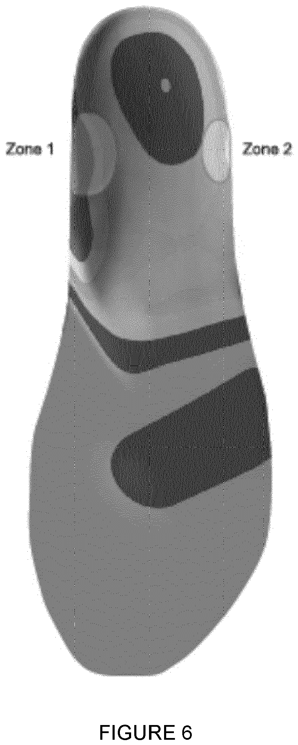

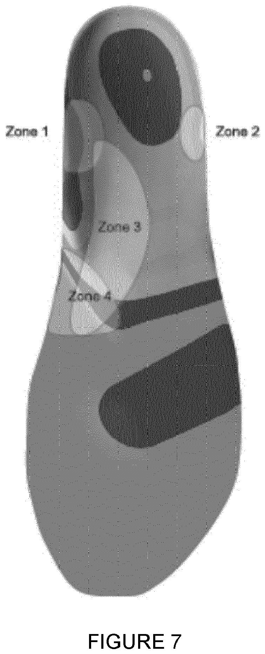

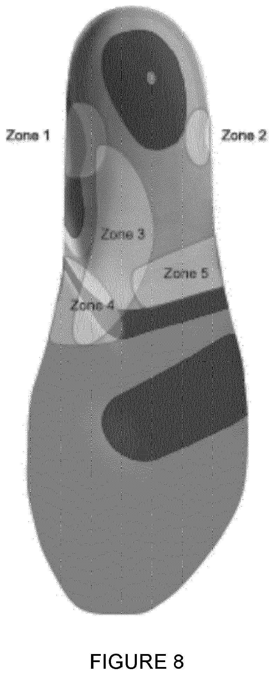

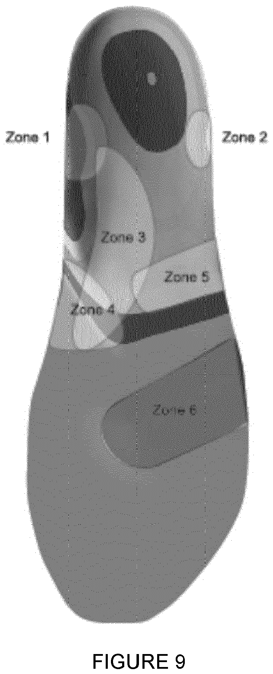

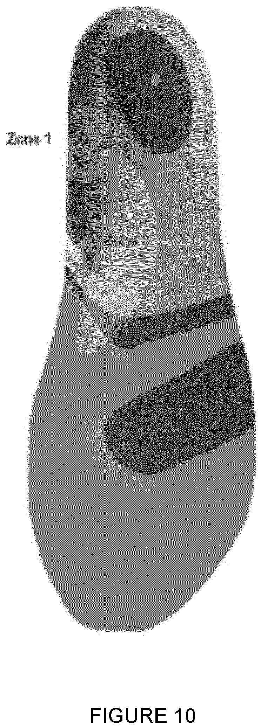

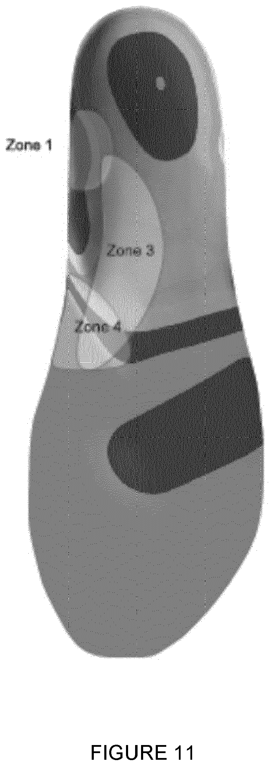

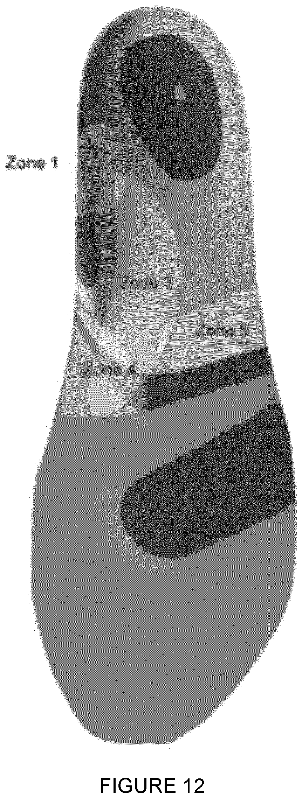

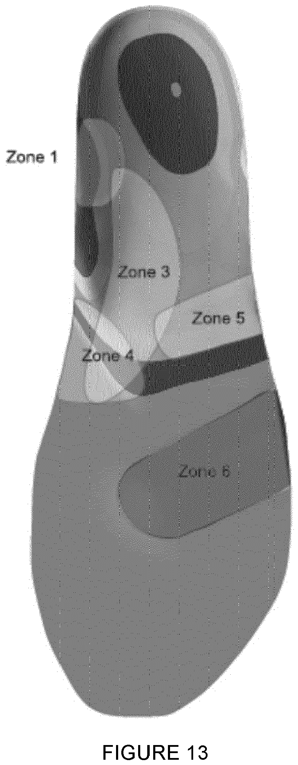

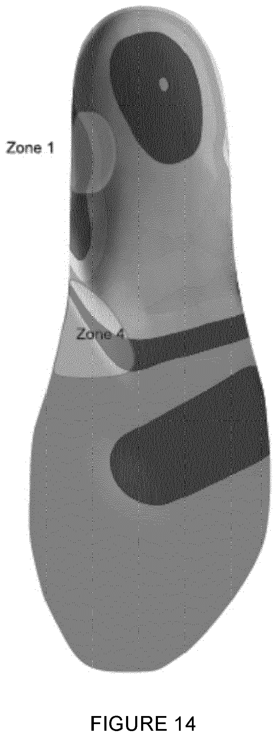

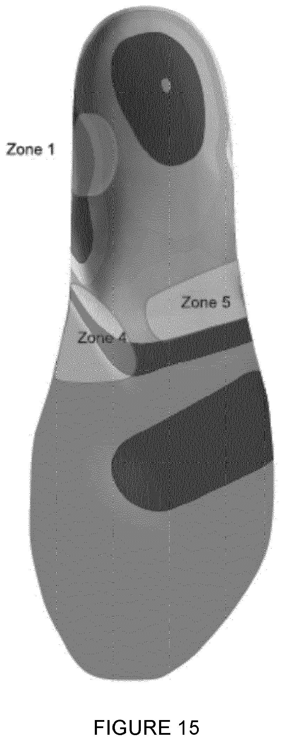

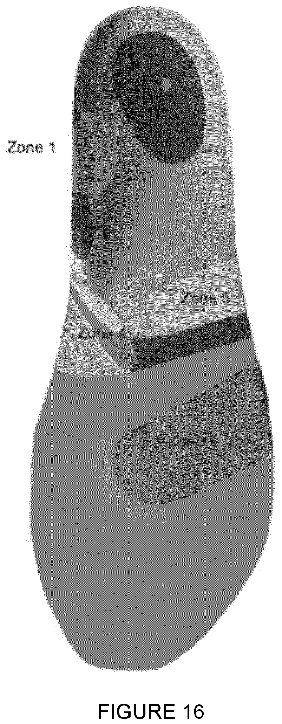

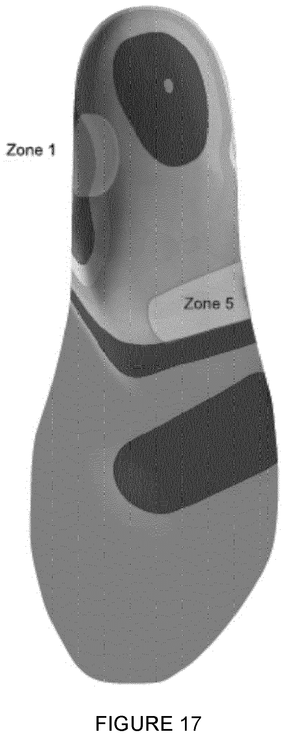

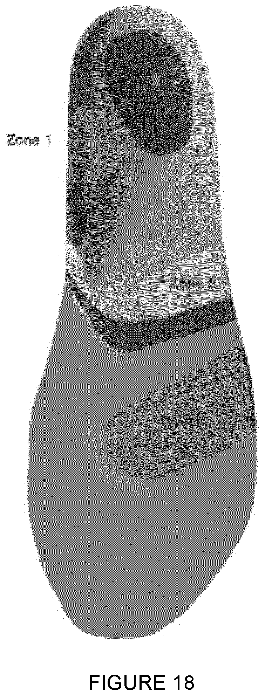

Alternatively, custom orthotics may be manufactured which include, amongst any other features suggested by the customised analysis, raised surfaces or grooves (as appropriate) in all or some of the above identified zones. For example, a patient may have a need for stimulation/blocking (as appropriate) in zones 7 only (Figure 4), zone 8 only (Figure 5), zones 1 and 2 only (Figure 6), zones 1 to 4 (Figure 7), zones 1 to 5 (Figure 8), zones 1 to 6 (Figure 9) zones 1 and 3 only (Figure 10), zones 1, 3 and 4 (Figure 11), zones 1, 3, 4 and 5 (Figure 12), zones 1, 3, 4, 5 and 6 (Figure 13), zones 1 and 4 only (Figure 14), zones 1, 4 and 5 (Figure 15), zones 1, 4, 5 and 6 (Figure 16), zones 1 and 5 only (Figure 17) or zones 1, 5 and 6 (Figure 18).

The orthotics shown in Figures 1, 2, 4 to 18 are all full length orthotics. However, inventive orthotics are less than half the length of the foot as shown in Figure 19, and do not include any of the zones toward the front of the orthotic shown in Figures 1, 2, 4-18.

The word 'comprising', and forms of the word 'comprising', when used in this specification is taken to specify the presence of stated features, integers, steps or components but does not preclude the presence or addition of one or more other features, integers, steps, components or groups thereof.

In this specification where a document, act or item of knowledge is referred to or discussed, this reference or discussion is not an admission that the document, act or item of knowledge or any combination thereof was at the priority date, publicly available, known to the public, part of the common general knowledge; or known to be relevant to an attempt to solve any problem with which this specification is concerned.

## Claims

1. An orthotic for a foot, having a top side and a bottom side, the orthotic being less than half the length of the foot, wherein the top side of the orthotic includes a first raised section (zone 1) located at a position corresponding to the medial side of the calcaneus of the foot and around the insertion site of the tibialis posterior into the side of the navicular of the foot, thereby increasing the support of the orthotic to facilitate the tibialis posterior muscle group, and
further comprising a second raised section (zone 2) in the top side of the orthotic located at a position corresponding to the cuboid area of the lateral side of the foot and extending anterior along one third of the shaft of the fifth metatarsal, thereby stimulating the peroneal muscle insertions and tendons that track through this area.

2. The orthotic according to claim 1, wherein the first raised section (zone 1) is a substantially semicircular arc with a diameter of 2 to 4 cm.

3. The orthotic according to claim 1 or 2, wherein the bottom side of the orthotic has no pitched heel or only a minimal pitched heel.

4. The orthotic according to any of claims 1 to 3, wherein a height of the first and/or second raised section (zone 1, zone 2) is greatest towards the centre of the respective zone.

5. The orthotic according to claim 4, wherein the height of the first and/or second raised section (zone 1, zone 2) is less than 25 mm.

6. The orthotic according to claim 5, wherein the height of the first and/or second raised section (zone 1, zone 2) is between 2 mm and 20 mm.

7. The orthotic according to claim 6, wherein the height of the first and/or second raised section (zone 1, zone 2)is between 3 mm and 8 mm.

## Patentansprüche

1. Orthese für einen Fuß mit einer Oberseite und einer Unterseite, wobei die Orthese weniger als die Hälfte der Länge des Fußes beträgt, wobei die Oberseite der Orthese einen ersten erhöhten Abschnitt (Zone 1) enthält, der sich an einer Position entsprechend der medialen Seite des Fersenbeins des Fußes und um die Einsetzstelle des Tibialis-Posterior in die Seite des Navikulars des Fußes befindet, wodurch die Unterstützung der Orthese erhöht wird, um die Tibialis-Posterior-Muskelgruppe zu erleichtern, und
ferner aufweisend einen zweiten erhöhten Abschnitt (Zone 2) in der Oberseite der Orthese, der sich an einer Position entsprechend dem Kuboidbereich der Außenseite des Fußes befindet und sich in der Vorderrichtung entlang eines Drittels des Schafts des fünften Mittelfußknochens erstreckt, wodurch die Peronäusmuskeleinsätze und -sehnen, die durch diesen Bereich verlaufen, stimuliert werden.

2. Orthese nach Anspruch 1, wobei der erste erhöhte Abschnitt (Zone 1) ein im Wesentlichen halbkreisförmiger Bogen mit einem Durchmesser von 2 bis 4 cm ist.

3. Orthese nach Anspruch 1 oder 2, wobei die Unterseite der Orthesen keinen geneigten Absatz oder nur einen minimal geneigten Absatz hat.

4. Orthese nach einem der Ansprüche 1 bis 3, wobei eine Höhe des ersten und/oder zweiten erhöhten Abschnitts (Zone 1, Zone 2) zur Mitte der jeweiligen Zone am größten ist.

5. Orthese nach Anspruch 4, wobei die Höhe des ersten und/oder zweiten erhöhten Abschnitts (Zone 1, Zone 2) weniger als 25 mm beträgt.

6. Orthese nach Anspruch 5, wobei die Höhe des ersten und/oder zweiten erhöhten Abschnitts (Zone 1, Zone 2) zwischen 2 mm und 20 mm beträgt.

7. Orthese nach Anspruch 6, wobei die Höhe des ersten und/oder zweiten erhöhten Abschnitts (Zone 1, Zone 2) zwischen 3 mm und 8 mm beträgt.

## Revendications

1. Orthèse pour un pied, ayant un côté supérieur et un côté inférieur, l'orthèse ayant une longueur inférieure à la moitié de celle du pied, le côté supérieur de l'orthèse incluant une première section surélevée (zone 1) qui est située à une position correspondant au côté médial du calcanéus du pied et autour du site d'insertion du tibial postérieur dans le côté du naviculaire du pied, ce qui augmente le support de l'orthèse en faveur du groupe musculaire comprenant le tibial postérieur, et
comprenant en outre une deuxième section surélevée (zone 2) dans le côté supérieur de l'orthèse qui est située à une position correspondant à la zone cuboïde du côté latéral du pied et qui s'étend vers l'avant le long d'un tiers de la tige du cinquième métatarsien, ce qui stimule les insertions et tendons musculaires péroniers qui traversent cette zone.

2. Orthèse selon la revendication 1, dans laquelle la première section surélevée (zone 1) est un arc sensiblement semi-circulaire avec un diamètre de 2 à 4 cm.

3. Orthèse selon la revendication 1 ou 2, dans laquelle le côté inférieur de l'orthèse n'a aucun talon incliné ou n'a qu'un talon incliné au minimum.

4. Orthèse selon l'une quelconque des revendications 1 à 3, dans laquelle la hauteur de la première et/ou deuxième section surélevée (zone 1, zone 2) est la plus grande vers le centre de la zone respective.

5. Orthèse selon la revendication 4, dans laquelle la hauteur de la première et/ou deuxième section surélevée (zone 1, zone 2) est inférieure à 25 mm.

6. Orthèse selon la revendication 5, dans laquelle la hauteur de la première et/ou deuxième section surélevée (zone 1, zone 2) est entre 2 mm et 20 mm.

7. Orthèse selon la revendication 6, dans laquelle la hauteur de la première et/ou deuxième section surélevée (zone 1, zone 2) est entre 3 mm et 8 mm.
